(19) Europäisches Patentamt
European Patent Office
Office européen des brevets

(11) EP 2 149 048 B1

(12) EUROPEAN PATENT SPECIFICATION

(45) Date of publication and mention
of the grant of the patent:
**07.11.2012 Bulletin 2012/45**

(51) Int Cl.:
***G01N 21/78*** (2006.01)       ***G01N 33/543*** (2006.01)
***G01N 33/538*** (2006.01)

(21) Application number: **08760265.2**

(22) Date of filing: **30.05.2008**

(86) International application number:
**PCT/EP2008/056675**

(87) International publication number:
**WO 2008/145722 (04.12.2008 Gazette 2008/49)**

(54) **DEVICE AND PROCESS FOR BINDING ASSAYS**

VORRICHTUNG UND VERFAHREN ZUR BINDUNG VON ASSAYS

DISPOSITIF ET PROCÉDÉ POUR ESSAIS DE LIAISON

(84) Designated Contracting States:
**AT BE BG CH CY CZ DE DK EE ES FI FR GB GR
HR HU IE IS IT LI LT LU LV MC MT NL NO PL PT
RO SE SI SK TR**

(30) Priority: **30.05.2007 DE 102007025311**

(43) Date of publication of application:
**03.02.2010 Bulletin 2010/05**

(73) Proprietor: **FZMB GmbH Forschungszentrum für
Medizintechnik
und Biotechnologie
99947 Bad Langensalza (DE)**

(72) Inventors:
• **MIETHE, Peter
06632 Schleberoda (DE)**
• **SÖFFING, Hans Hermann
99423 Weimar (DE)**

(74) Representative: **von Kreisler Selting Werner
Deichmannhaus am Dom
Bahnhofsvorplatz 1
50667 Köln (DE)**

(56) References cited:
**EP-A- 0 021 214      WO-A-87/07384
WO-A-95/19569      DE-A1-102004 006 470**

**Description**

The present invention relates to a process and a device for performing quantitative affinity-chromatographical quick tests with photometric evaluation with a porous filler material having a reflecting inner surface and receptors immobilized thereon and being provided in a container having an inlet and outlet.

Affinity-chromatographical quick tests are based on biospecific interactions of ligands and receptors. Typically, the receptor is immobilized on a surface of a solid while the ligand to be assayed is supplied via a liquid phase and is specifically bound to the surface. A usual construction form for quick tests is described in U.S. Patent No. 4,943,522. Such quick tests are employed, for example, as pregnancy test strips. They are prepared on the basis of nitrocellulose strips which are capped by a glass fiber membrane at the upper portion thereof. The sample is applied to the upper portion of the glass fiber membrane and reacts, while it migrates through the non-woven material, with the conjugate incorporated therein. As a rule, these are antibody-gold particles. The immunocomplex formed migrates further through the nitrocellulose membrane and is bound to capture antibodies immobilized thereon. As a result, a stained band is formed. In the past, there have been numerous attempts to read out such test strips quantitatively by either reflectometry or transmittance measurement. However, the sensitivity and precision that can be achieved thereby is relatively poor, and the dynamic range of such measurements is limited to 2-2.5 orders of magnitude.

Another quick test method is described in EP-A-0 557 288, EP-0 634 015 or WO-A-95/19569. It is based on a flow column containing porous material with an immobilized receptor. The binding of an analyte is effected while flowing through the column. The bound analyte can subsequently be visualized by adding a second, labeled, receptor. The use of an enzyme-antibody conjugate and subsequent addition of precipitating tetramethylbenzidine has proven advantageous. In this case, a blue precipitate forms that can be detected quantitatively by analyzing the transmitted light directly by transmittance measurement on the porous material. A particularly high sensitivity and low detection limits are achieved thereby if a measuring cuvette according to DE 102004 006470A1 is employed. Such a procedure is described in Gessler et al., 2005 Appl. Environ. Microbiol. (2005) p7897-7903. This method is dearly superior to the above described membrane method in terms of sensitivity, but likewise it also exhibits a very narrow dynamic range of 2.5 orders of magnitude at most. In the method described, scattered light measurements show a behavior that is comparable in principle.

WO-A-87/07384 discloses a device for concentrating enzyme immunoassay sandwich product in a very narrow band in a column for development and visual reading. The device may be constructed using a clear or transparent vessel or column, with a fluid inlet, in which there is disposed a band or a plurality of bands of small particles to which one of an immunochemical couple is bound, the other member of which couple is the species to be determined. The immunochemical band is defined by bands of non-immunochemically active particles. An absorbent pad may support the bands.

EP-A-0021214 discloses a specific binding assay method, and a test device and test kit for use therein, for determining a ligand, such as an antigen or antibody, in, or the ligand binding capacity of, a liquid medium, particularly a body fluid such as serum, wherein the unknown ligand competes with a labeled component, such as a radiolabeled form of the ligand or of a binding analog of the ligand, for binding with a binding partner, and wherein separation of the resulting bound-species and free-species of the labeled component is accomplished by allowing the liquid reaction mixture to be drawn by capillary action into a column comprising a bed of an adsorbent material selective for one of the two species. The improvement comprises using a column containing at least one additional bed of capillarily absorbent material disposed above said absorbent bed and which is substantially nonadsorbent relative to the one of said two species which said adsorbent bed selectively binds. The use of specific types of capillarily absorbent beds in addition to the selectively absorbent bed to form multilayer columns overcomes several unforeseen problems encountered in using the previously known column chromatography method for certain assay applications.

Alternative reading-out techniques, such as fluorometry and luminometry, have a better dynamic range of up to 4 orders of magnitude. Generally, however, the necessary measuring technology is relatively tedious and thus expensive in these methods. In addition, interference may occur from quenching and bleaching effects. Therefore, in contrast to photometry, these techniques are not suitable for the preparation of simple and inexpensive measuring systems.

With analytes occurring in a broader range of concentrations, the narrow dynamic range of the photometric methods in turn has the result that several dilutions of the solution to be analyzed would have to be prepared and examined for one analysis. This procedure is not user-friendly and has the consequence that the quick test method cannot be employed.

It was the object of the invention to provide a process and a corresponding device which allow analytes to be assayed quickly and precisely by affinity chromatography in a broad dynamic range on the basis of a simple, inexpensive optical transmission technique.

The above object is achieved by a device according to claim 1 and a process according to claim 10. The dependent claims relate to particular embodiments of the device according to the invention.
(Appl. Environ. Microbiol. (2005) p7897-7903)

The device according to the invention is suitable for performing quantitative affinity-chromatographical quick tests with photometric evaluation. The device includes at least one filter element 2 formed from a porous filler material 5 on which receptors are immobilized and which is provided in a container 1 having an inlet 3 and an outlet 4. The filter element 2 has a reflecting inner surface. The device according to the invention is characterized in that:

- the porous filler material 5 is provided between two liquid-permeable separator elements 6 to form a column-shaped segment 8;

- at least two column-shaped segments 8 are provided in the device;

- the receptors are in a specific concentration within a range of from 1 ng/ml to 200 g/ml of filter volume;

- the separator elements 6 have reflecting properties on the surface adjacent to the porous filler material;

- the separator element (6) is a porous filter element 2 having a mean pore diameter of from 1 to 100 $\mu$m and a pore volume fraction of from 20% to 80%;

- the device has such a design as to allow the device to be flowed through by a solution containing analytes that can be bound to receptors immobilized at the surface of the porous filler materials at a rate within a range of from 1.5 ml/min to 0.01 ml/min;

- the device has a means 10 with analytes bound to receptors immobilized at the surface of the porous filler materials that is quantified on one or more segments 8 by light transmittance measurement or/and light scattering measurement at an angle of between 180° and 90° either directly of upon addition of one or more secondary reagents undergoing specific binding with the analytes to produce a color directly or indirectly;

- wherein the device has an evaluation unit that allows the amount of analyte to be assayed by means of a calibration curve taking into account the transmittance or values derived therefrom of at least two segments.

In one embodiment, the segments 8 containing the porous filler material have a length of from 0.5 to 10 mm and a diameter of from 0.5 to 5 mm.

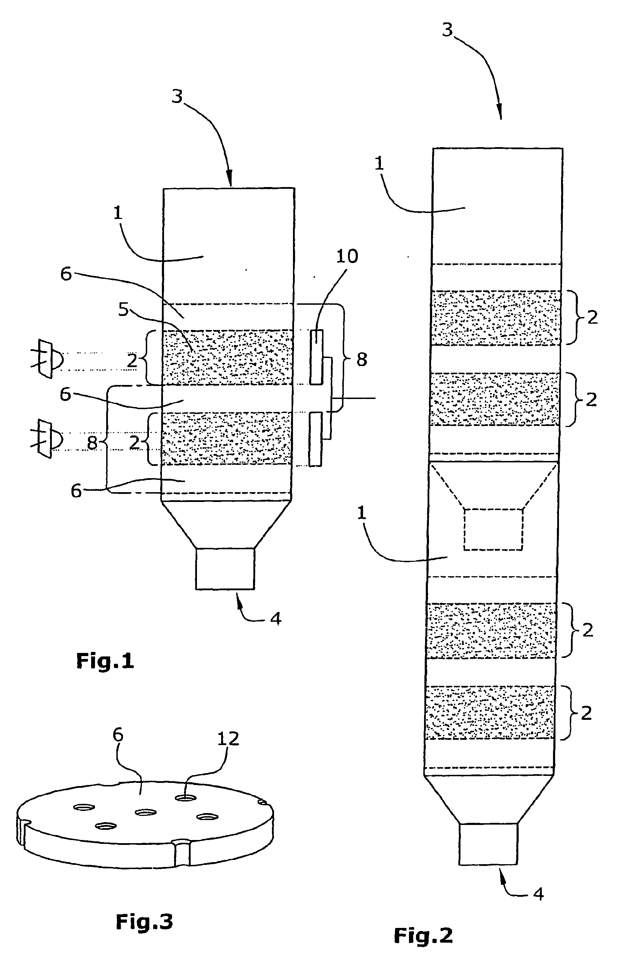

Figure 1 shows a schematic representation of the device according to the invention.

It may be advantageous if at least two containers are connected with one another (Figure 2). The outlet of one container may be connected to the inlet of said at least second container detachably, for example, by insertion.

In a further embodiment of the device according to the invention, the evaluation unit has a photodiode/light-emitting diode assembly for establishing the transmittance that can be designed as light-emitting diodes and a charge-coupled device (CCD) line or CCD array.

A spectrometric evaluation of transmittance can also be performed.

For detecting the analyte, staining by a receptor/particle conjugate and/or receptor/enzyme conjugate with subsequent addition of an enzyme substrate may be effected.

In another embodiment of the device according to the invention, the receptor concentration in the segments increases from the inlet 3 to the outlet 4.

The present invention also relates to a process for performing quantitative affinity-chromatographical quick tests for the presence of analytes using photometric evaluation wherein a solution containing or suspected to contain an analyte is introduced through the inlet 3 of container 1 of the device according to the invention and contacted with the filler material in segments 8 and, following adsorption of the analyte to receptors having affinity for said analyte, the presence of the analyte bound to the receptors is assayed quantitatively and/or qualitatively.

The invention is described in more detail in the following.

We explicitly proceed from the process described above on the basis of a photometric measuring cuvette with a heterogeneous porous filler material. The porous filler material is preferably employed in the form of filter segments on the inner surface of which receptors are immobilized. According to the invention, at least two of these elements may be arranged in succession in one tube and separated by liquid-permeable separator elements having reflecting properties on the interface towards the filter element (Figure 1). The linear arrangement is flowed through successively by the solution containing the analyte (ligand) to be determined.

Figure 1 shows a separator disk 6 having one or two reflecting major surfaces.

In particular, the inserted filter elements 2 (Figure 1) have a reflecting inner surface. The mean pore diameters should be within a range of from 1 $\mu$m to 100 $\mu$m, and the pore volume fraction is to be from 20% to 80%.

In one embodiment according to the invention, column-shaped receptor-containing filter elements 2 having a length within a range of from 0.5 to 5 mm and a diameter within a range of from 0.5 to 5 mm are employed. The separator elements 6 have the same diameters, but a lower thickness within a range of from 10 $\mu$m to 2.5 mm. Generally, each receptor-containing filter element 2 is bounded by two separator elements 6 on the top and bottom thereof. In a simple embodiment according to the invention, the following sequence is obtained:

separator element 6/active filter element 2/separator element 6/active filter element 2/separator element 6.

In a further embodiment of the device according to the invention in the form of an analytical tube, a substantially higher number of filter elements 2 and separator elements 6 may be arranged in succession. Through the linear arrangement that can be flowed through, the solution containing the ligand to be assayed can flow at a rate of from 1.5 ml/min to 0.01 ml/min with a correspondingly sized metering device or by hydrostatic pressure alone, wherein the specific concentration of the receptor on the active receptor-containing filter elements should be selected within a range of from 2 $\mu$g/ml to 200 mg/ml of filter volume.

The quantification of the bound ligand is effected separately on the successively arranged filter elements 2 in a per se known manner. For this purpose, one or more per se known marker reagents, such as immuno, gold, antibody, stain conjugates, antibody-enzyme conjugates etc. that react with the ligand and form a readily detectable, especially by optical detection, complex or catalyze the formation of a detectable colored matter may usually be additionally added before the measurement. According to the invention, the change in transmittance of or the light scattered at a defined angle from the individual filter elements is recorded. Optical densities can be calculated therefrom on the basis of the Lambert-Beer law. Namely:

$$\lg (I_0/I) = OD = \varepsilon \, d_{\text{eff}} \, c$$

where:

OD = optical density
$\varepsilon$ = extinction coefficient
$d_{\text{eff}}$ = effective layer thickness, which depends on the light-scattering properties of the filters;
c = concentration of the analyte to be assayed;
$I_0$ = transmittance before the filter element is stained with the labeling substance;
I = transmittance after the filter element has been stained with the labeling substance.

According to the invention, the intensity values of the transmitted or scattered light of values derived therefrom, especially the optical densities calculated according to the stated equation, of at least two successive filter segments 2 are determined for calibration. Subsequently, derived measuring values, which are employed for establishing the calibration, are produced by combining these values on the basis of a suitable mathematical function. A simple form of combination is the summation or conversion to mean values of the OD values from the two filters.

It has been found that a calibration function having a broader dynamic range than that obtained from the evaluation of a single filter under comparable reaction conditions can be generated by plotting these derived values against the concentration.

Thus, an extension of the measuring range of up to 4 powers of ten, i.e., dynamic ranges of up to 6 powers of ten, in concentration can be realized. To achieve this, more than two filter segments 2 should usually be provided in succession, wherein such filter segments can have a very thin design. Typical measuring set-ups for the quantification of the transmitted or scattered light are photodiodes/light-emitting diodes assemblies or light-emitting diode cells in combination with CCD lines or CCD arrays.

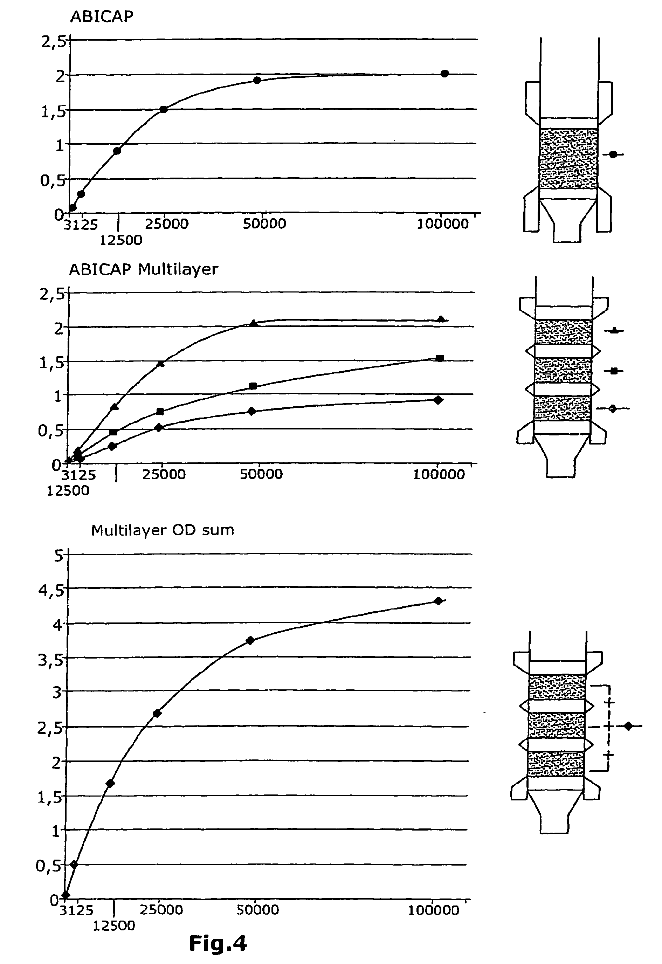

The procedure described is illustrated in Figure 4 taking a three-layered filter set-up as an example in comparison with a one-layered conventional set-up. In the example shown, the volume of the individual reference filter corresponds to the sum of the filter volumes of the three smaller filters in the multifilter set-up. The Figure is based on the data from Example 1.

Example 2 shows for an analogous three-layered set-up that very good coefficients of variation and thus a precision that is clearly superior to that of the conventional set-up can also be achieved with this process.

In the case of Example 3, the optical detection was effected with a CCD array. The Example shows at the same time that it is possible to arrange several segment assemblies having different specificities in succession and thus to perform multiplex analyses.

In the Example of Figure 4, a set-up is shown in which the receptor concentration on the filter segments increases from the inlet to the outlet. In this case, a relatively uniform color along the segment column whose length correlates with the analyte concentration is to be noted.

Figure 4: Photometric calibration curves for the immunological determination of *F*. *tularensis* on a porous filter element with immobilized anti-*F*. *tularensis* antibody and peroxidase/TMB detection (heterogeneous immunoassay according to Example 1).

Top: Calibration curve and optical set-up of a conventional measuring assembly based on a 5 x 5 mm polyethylene filter element and a single-beam photometer.

Middle: Calibration curves and optical set-up for an assembly proposed according to the invention of three filter elements of 1.6 x 5 mm separated by separator elements and the corresponding photometric three-layer set-up; the calibration curves were respectively established on the filter elements and may be used singly or on the basis of suitable mathematical combinations for evaluating the measurement.

Bottom: Summed calibration curve calculated from the 3 calibration curves for the *F. tularensis* determination on the set-up proposed according to the invention.

Example 1. Detection of LPS from *Franciscella tularensis:*

Sintered parts consisting of High Density Polyethylene (HDPE) with a mean pore diameter of 40 $\mu$m and a pore volume fraction of 50% in the dimensions (diameter $\times$ length) $5 \times 5$ mm (type 1) or 5 x 1.65 mm (type 2) on which 9 $\mu$g or 3 $\mu$g each, respectively, of monoclonal anti-*Francescella tularensis* antibodies was immobilized were employed. Analogous filter elements in the dimensions 5 x 0.5 mm (type 3) without binding activity (block filters) were employed as porous separator elements.

By inserting a type 1 filter element into microcolumns (d = 5 mm, V = 750 $\mu$l), an assembly corresponding to the scheme on the top of Figure 2 was prepared. This one-layered assembly corresponds to the above described prior art and served as a reference system.

The preparation of the multilayered assembly proposed according to the invention in accordance with the middle and bottom of Figure 2 was effected by sequential insertion according to the scheme:

Filter type 3/type 2/type 3/type 2/type 3/type 2/type 3.

*F. tularensis* extracted lipopolysaccharide was used as the control material. The concentration was stated on the basis of the germ numbers in cfu/ml before the extraction. The assays were performed in pipetting racks with a zero control and 6 calibrating samples each in duplicate. The addition of the sample and of all the subsequent reagents was effected directly into the inlet of the microcolumn. The flow rates were within a range of from 250 to 300 $\mu$l/min. After the binding of the sample (500 $\mu$l), an immune complex was formed on the column by adding anti-*F. tularensis*-POD conjugate and detected by precipitating TMB and subsequently quantified by transmittance measurement. The assay scheme is as follows:

- 500 $\mu$l sample in pphosphate buffered saline (PBS) buffer
- incubation for 4 min
- 500 $\mu$l of anti-*F. tularensis*-poly-HRP conjugate (8 $\mu$g/ml)
- incubation for 4 min
- 2 x 750 $\mu$l PBS buffer
- determination of transmittance $I_0$
- 500 $\mu$l of TMB substrate solution precipitating
- incubation for 6 min
- 500 $\mu$l of PBS buffer
- determination of transmittance I

The optical read-out of the reference columns was effected with a miniphotometer (SENOVA Cat. No. 30010001RDR). It is capable of realizing transmittance measurements on one-layered columns at a wavelength of 532 nm and an aperture diameter of 3.6 mm. The multilayer columns were measured with a modified photometer of the same fundamental design. It had a modified measuring head with 3 beam paths (aperture diameter 1.2 mm), each on the level of the active filter elements.

From the measured transmittance values, the OD values were calculated on the basis of formula 1. These OD values and the OD mean values calculated therefrom were shown as a function of the LPS concentration in Figure 4. The upper part shows the calibration curve of the one-layered system, the middle part shows the calibration of the individual filter layers, and the bottom part shows the calibration on the basis of the summed OD values.

Example 2: Detection of *Streptococcus mutans*

Sintered parts consisting of HDPE with a mean pore diameter of 40 $\mu$m and a pore volume fraction of 50% in the dimensions (diameter $\times$ length) 5 $\times$ 5 mm (type 1) or 5 x 1.65 mm (type 2) on which 15 $\mu$g or 5 $\mu$g each, respectively, of polyclonal affinity-purified serum from rabbit against *Streptococcus mutans* was immobilized were employed.

Disks according to Figure 3 were employed as separator elements. They had a metallic surface with partially reflecting properties. In addition, block filters according to type 3 in Example 1 were employed.

By inserting a type 1 filter element into microcolumns (d = 5 mm, V = 750 $\mu$l), an assembly corresponding to the scheme of Figure 4 (top) was prepared. This one-layered assembly corresponds to the above described prior art and served as a reference system. The preparation of the multilayered assembly proposed according to the invention in accordance with the middle and bottom of Figure 4 was effected by sequential insertion according to the scheme:

Type 3/type 2/disk/type 2'/disk/type 2/type 3'.

Cell culture material of S. *mutans* was used as the control material. The concentration in cfu/ml was determined by plating. The assays were performed in pipetting racks with a zero control and 6 calibrating samples each in duplicate. The addition of the sample and of all the subsequent reagents was effected directly into the inlet of the microcolumn. The flow rates were within a range of from 180 to 200 $\mu$l/min. After the binding of the sample (500 $\mu$l), an immune complex was formed on the column by adding affinity-purified biotinylated anti-*S. mutans* serum (rabbit). Subsequently, streptavidin-magnetic particles (0.5 to 1 $\mu$m, Chemagen GmbH, Baesweiler, Germany) were added for optical detection. The magnet particles can be well detected on the filters by photometry by transmittance measurement at 525 nm. The assay scheme is as follows:

- 500 $\mu$l sample
- incubation for 4 min
- 500 $\mu$l of anti-*S mutans*-biotin (Immunoglobulin G IgG fraction 8 $\mu$g/ml)
- incubation for 4 min
- determination of transmittance $I_0$
- 500 $\mu$l of streptavidin-magnetic particles (Chemagen)
- 750 $\mu$l of PBS buffer
- determination of transmittance I*

The determination of the transmittance values was effected as described in Example 1. The following Table 1 shows the obtained calibration data as mean values.

Table 1: Calibration values (mean values from duplicate determinations) for the S. mutans test on a one-layered filter (type 1) and the three-layered filter assembly and the sums and mean values calculated therefrom

| cfu/ml | OD one layer | OD filter 1 | OD filter 2 | OD filter 3 | sum | mean value |
|---|---|---|---|---|---|---|
| 0 | 0.53 | 0.036 | 0.037 | 0.033 | 0.106 | 0.035 |
| $1.00 \cdot 10^3$ | 0.097 | 0.088 | 0.061 | 0.039 | 0.188 | 0.063 |
| $1.00 \cdot 10^4$ | 0.177 | 0.184 | 0.11 | 0.42 | 0.714 | 0.238 |
| $1.00 \cdot 10^5$ | 0.586 | 0.552 | 0.411 | 0.214 | 1.177 | 0.392 |
| $1.00 \cdot 10^6$ | 1.129 | 1.022 | 0.673 | 0.341 | 2.036 | 0.679 |
| $1.00 \cdot 10^7$ | 1.86 | 1.682 | 1.249 | 0.663 | 3.594 | 1.198 |
| $1.00 \cdot 10^8$ | 2.0025 | 1.806 | 1.601 | 1.002 | 4.409 | 1.470 |
| $1.00 \cdot 10^9$ | 2.0965 | 1.917 | 1.722 | 1.239 | 4.878 | 1.626 |

Example 3: Simultaneous assay of *F. tularensis* and *Y. pestis*

Sintered parts consisting of HDPE with a mean pore diameter of 12 $\mu$m and a pore volume fraction of 40% in the dimensions (diameter x length) 2.5 x 1.6 mm were employed. Some of the filters were coated with 2 $\mu$g of monoclonal antibody against *F. tularensis* (type 1"), and some of the filters were coated with 2 $\mu$g of monoclonal antibody against the F1 antigen of *Y. pestis* (type 2"). The separator elements were prepared from 0.15 mm thick aluminum foil that was provided with 3 holes each (d = 0.2 mm). In addition, block filters in the dimensions 2.5 mm x 1.6 mm were employed.

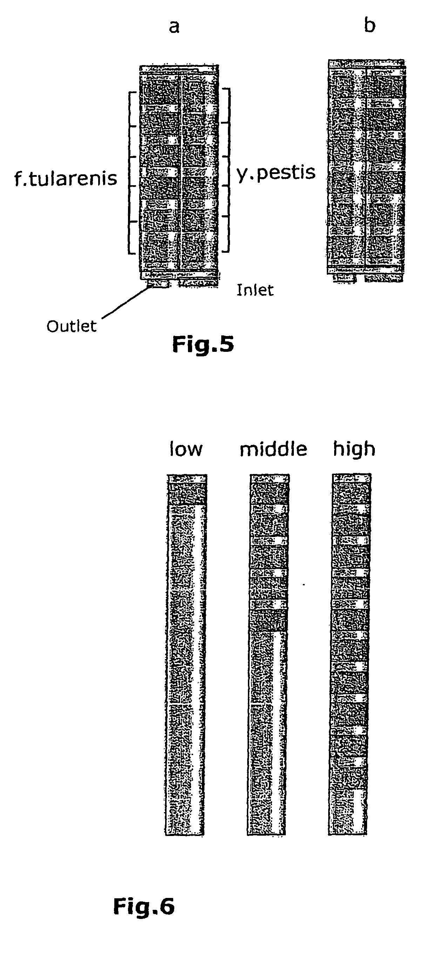

A multilayer assembly corresponding to Figure 5 and based on 2 parallel tubes connected through a channel was prepared. The sample (500 µl) was preincubated with 4 µg of anti-*F. tularensis*-POD conjugate (POD = β-peroxidase) and 3 µg of antl-*Y. pestis*-POD conjugate for 6 minutes. Subsequently, metering with a syringe pump was effected at as flow rate of 1.2 ml/min. Subsequently, 1.5 ml of PBS buffer was metered through a second channel of the syringe pump in 5 seconds, followed by metering 500 µl of precipitating TMB through a third channel in 3 seconds. After an incubation time of 4 minutes, PBS buffer (500 µl) was again metered through channel 2. Subsequently, the optical evaluation was performed in the CCD array assembly as shown in Figure 5.

In an exemplary manner, Figure 5 shows the result in a schematic form for an analysis with a high (5000 cfu/ml) *F. tularensis* concentration and iow *Y. pestis* concentration (0.2 ng/ml) and the converse case of a low *F. tularensis* (425 cfu/ml) and high *Y. pestis* concentration (10 ng/ml). The calibrations obtained for the two analytes on the basis of the sums of the calculated OD values of the 6 filters are shown in Figure 6.

Example 4: Quantification of *Botulinus* toxin A by the number of stained segments

Eleven sintered parts consisting of HDPE with a mean pore diameter of 12 µm and a pore volume fraction of 40% in the dimensions (diameter x length) 1 x 1.6 mm were employed.

The filters were coated with 7.5 µg of monoclonal antibody against *Botulinus* toxin A. The separator elements were prepared from 0.15 mm thick aluminum foil that was provided with 3 holes each (d = 0.2 mm).

A multilayer assembly corresponding to Figure 6 was prepared.

The sample (900 µl) was preincubated with 4 µg of biotinylated anti-BotA antibody for 6 minutes. Subsequently, the metering was effected by feeding onto the column at a flow rate of about 0.4 ml/min. Subsequently, 500 µl of SA-polyHRP (HRP = horse raddish peroxidase) was pipetted, the column was washed twice with 1.5 ml of PBS buffer after 6 minutes, followed by metering 500 µl of precipitating TMBN in about 1.25 minutes. After an incubation time of 6 minutes, 1 ml of PBS buffer was again metered. Subsequently, the optical evaluation was performed by a CCD array assembly.

In an exemplary manner, Figure 6 shows the result in a schematic form for an analysis with a low (0.5 ng/ml) and high (5 ng/ml) concentration. The number of stained filters directly indicates the detected concentration in the sample.

**Claims**

1.   A device for performing quantitative affinity-chromatographical quick tests with photometric evaluation including at least one filter element (2) formed from a porous filler material (5) on which receptors are immobilized and which is provided in a container (1) having an inlet (3) and an outlet (4), said filter element (2) having a reflecting inner surface, **characterized in that**:

   - the porous filler material (5) is provided between two liquid-permeable separator elements (6) to form a column-shaped segment (8);
   - at least two column-shaped segments (8) are provided in the device;
   - the receptors are in a specific concentration within a range of from 1 ng/ml to 200 g/ml of filter volume;
   - the separator elements (6) have reflecting properties on the surface adjacent to the porous filler material;
   - the separator element (6) is a porous filter element having a mean pore diameter of from 1 to 100 µm and a pore volume fraction of from 20% to 80%;
   - the device has such a design as to allow the device to be flowed through by a solution containing analytes that can be bound to receptors immobilized at the surface of the porous filler materials at a rate within a range of from 1.5 ml/min to 0.01 ml/min;
   - the device has a means (10) with which analytes bound to receptors immobilized at the surface of the porous filler materials are quantified on one or more segments (8) by light transmittance measurement or/and light scattering measurement at an angle of between 180° and 90° either directly or upon addition of one or more reagents which specifically bind with the analytes to produce a color directly or indirectly;
   - wherein the device has an evaluation unit that allows the amount of analyte to be assayed by means of a calibration curve taking into account the transmittance or values derived therefrom of at least two segments.

2.   The device according to claim 1, **characterized in that** the segments (8) containing the porous filler material have a length of from 0.5 to 10 mm and a diameter of from 0.5 to 5 mm.

3.   The device according to claim 1 and/or 2, **characterized in that** at least two devices are connected with one another.

4. The device according to at least one of claims 1 to 3, **characterized in that** the evaluation unit has a photodiode/ light-emitting diode assembly for establishing the transmittance.

5. The device according to at least one of claims 1 to 4, **characterized in that** the evaluation unit has an assembly of light-emitting diodes and a CCD line or CCD array for establishing the transmittance.

6. The device according to at least one of claims 1 to 5, **characterized in that** the staining is effected by a receptor-particle conjugate.

7. The device according to at least one of claims 1 to 6, **characterized in that** the staining is effected by a receptor-enzyme conjugate followed by adding an enzyme substrate.

8. The device according to claims 1 to 7, **characterized in that** the receptor concentration in the segments increases from the inlet (3) to the outlet (4).

9. A process for performing quantitative affinity-chromatographical quick tests for the presence of analytes using photometric evaluation wherein a solution containing or suspected to contain an analyte is introduced through the inlet of the device according to any of claims 1 to 8 and contacted with the porous filler material in segments (8) and, following adsorption of the analyte to receptors having affinity for said analyte, the presence of the analyte bound to the receptors is assayed quantitatively and/or qualitatively.

**Patentansprüche**

1. Vorrichtung zur Durchführung quantitativer affinitätschromatographischer Schnelltests mit photometrischer Auswertung, umfassend wenigstens ein Filterelement (2), das aus einem porösen Füllstoff (5) gebildet ist und auf dem Rezeptoren immobilisiert sind und das sich in einem Behälter (1) befindet, der einen Einlass (3) und einen Auslass (4) aufweist, wobei das Filterelement (2) eine reflektierende Innenfläche aufweist, **dadurch gekennzeichnet, dass**:

   - sich der poröse Füllstoff (5) zwischen zwei flüssigkeitsdurchlässigen Trennelementen (6) befindet, wobei ein säulenförmiges Segment (8) entsteht;
   - sich wenigstens zwei säulenförmige Segmente (8) in der Vorrichtung befinden;
   - die Rezeptoren in einer spezifischen Konzentration im Bereich von 1 ng/ml bis 200 g/ml Filtervolumen vorliegen;
   - die Trennelemente (6) reflektierende Eigenschaften auf der an den porösen Füllstoff angrenzenden Fläche aufweisen;
   - das Trennelement (6) ein poröses Filterelement ist, das einen mittleren Porendurchmesser von 1 bis 100 $\mu$m und einen Porenvolumenanteil von 20% bis 80% aufweist;
   - die Vorrichtung einen solchen Aufbau hat, dass die Vorrichtung von einer Lösung, die Analyte enthält, welche an Rezeptoren, die auf der Oberfläche der porösen Füllstoffe immobilisiert sind, gebunden werden können, mit einer Geschwindigkeit im Bereich von 1,5 ml/min bis 0,01 ml/min durchflossen werden kann;
   - die Vorrichtung eine Einrichtung (10) aufweist, mit der Analyten, die an auf der Oberfläche der porösen Füllstoffe immobilisierte Rezeptoren gebunden sind, auf einem oder mehreren Segmenten (8) durch Lichttransmissionsmessung oder/und Lichtstreuungsmessung unter einem Winkel zwischen 180° und 90° entweder direkt oder nach Zugabe von einem oder mehreren Reagentien, die die Analyten spezifisch unter direkter oder indirekter Bildung einer Farbe binden, quantifiziert werden;
   - wobei die Vorrichtung eine Auswertungseinheit aufweist, die die Messung der Menge des Analyten mittels einer Eichkurve ermöglicht, wobei die Transmission oder davon abgeleitete Werte von wenigstens zwei Segmenten berücksichtigt werden.

2. Vorrichtung gemäß Anspruch 1, **dadurch gekennzeichnet, dass** die Segmente (8), die den porösen Füllstoff enthalten, eine Länge von 0,5 bis 10 mm und einen Durchmesser von 0,5 bis 5 mm aufweisen.

3. Vorrichtung gemäß Anspruch 1 und/oder 2, **dadurch gekennzeichnet, dass** wenigstens zwei Vorrichtungen miteinander verbunden sind.

4. Vorrichtung gemäß wenigstens einem der Ansprüche 1 bis 3, **dadurch gekennzeichnet, dass** die Auswertungseinheit eine Photodioden/LED-Baugruppe aufweist, um die Transmission zu bestimmen.

**5.** Vorrichtung gemäß wenigstens einem der Ansprüche 1 bis 4, **dadurch gekennzeichnet, dass** die Auswertungseinheit eine Baugruppe aus LEDS und einer CCD-Zeile oder einem CCD-Feld aufweist, um die Transmission zu bestimmen.

**6.** Vorrichtung gemäß wenigstens einem der Ansprüche 1 bis 5, **dadurch gekennzeichnet, dass** die Färbung durch ein Rezeptor-Teilchen-Konjugat erfolgt.

**7.** Vorrichtung gemäß wenigstens einem der Ansprüche 1 bis 6, **dadurch gekennzeichnet, dass** die Färbung durch ein Rezeptor-Enzym-Konjugat und anschließendes Hinzufügen eines Enzymsubstrats erfolgt.

**8.** Vorrichtung gemäß den Ansprüchen 1 bis 7, **dadurch gekennzeichnet, dass** die Rezeptorkonzentration in den Segmenten vom Einlass (3) zum Auslass (4) zunimmt.

**9.** Verfahren zur Durchführung quantitativer affinitätschromatographischer Schnelltests auf die Anwesenheit von Analyten mit Hilfe von photometrischer Auswertung, wobei eine Lösung, die einen Analyten enthält oder von der man annimmt, dass sie einen Analyten enthalten könnte, durch den Einlass der Vorrichtung gemäß einem der Ansprüche 1 bis 8 eingeführt und mit dem porösen Füllstoff in den Segmenten (8) in Kontakt gebracht wird und nach Adsorption des Analyten an Rezeptoren, die eine Affinität zu dem Analyten aufweisen, die Gegenwart des an die Rezeptoren gebundenen Analyten quantitativ und/oder qualitativ bestimmt wird.


**Revendications**

**1.** Dispositif pour exécuter des essais quantitatifs rapides par affinité chromatographique avec une évaluation photométrique comportant au moins un élément de filtration (2) formé à partir d'un matériau de remplissage poreux (5) sur lequel des récepteurs sont immobilisés et qui est fourni dans un conteneur (1) présentant un orifice d'entrée (3) et un orifice de sortie (4), ledit élément de filtration (2) présentant une surface interne réfléchissante, **caractérisé en ce que** :

   - le matériau de remplissage poreux (5) est fourni entre deux éléments de séparation perméables au liquide (6) en vue de former un segment configuré en forme de colonne (8) ;
   - au moins deux segments configurés en colonne (8) sont prévus dans le dispositif ;
   - les récepteurs se trouvent dans une concentration spécifique se situant dans une plage allant de 1 ng/ml à 200g/ml de volume de filtration ;
   - les éléments de séparation (6) ont des propriétés réfléchissantes sur la surface adjacente au matériau de remplissage poreux ;
   - l'élément de séparation (6) est un élément de filtration poreux présentant un diamètre moyen de pore allant de 1 à 100 $\mu$m et une fraction de volume de pore allant de 20% à 80% ;
   - le dispositif présente une conception telle qu'elle permette au dispositif d'être traversé par une solution contenant des éléments à analyser qui peuvent être liés aux récepteurs immobilisés au niveau de la surface des matériaux de remplissage poreux à une vitesse se situant dans une plage allant de 1,5 ml/min à 0,01 ml/min ;
   - le dispositif comporte des moyens (10) à l'aide desquels les éléments à analyser liés aux récepteurs immobilisés au niveau de la surface des matériaux de remplissage poreux sont quantifiés sur un ou plusieurs segment(s) (8) par une mesure de transmission de la lumière ou/et une mesure de diffusion de la lumière suivant un angle compris entre 180° et 90°, soit directement , soit lors de l'ajout d'un ou de plusieurs réactif(s) qui se lie(nt) de façon spécifique avec les éléments à analyser afin de produire une couleur directement ou indirectement ;
   - dans lequel le dispositif comporte une unité d'évaluation qui permet à la quantité d'éléments à analyser d'être mesurée au moyen d'une courbe de calibrage prenant en compte le facteur de transmission ou des valeurs en sont déduites d'au moins deux segments.

**2.** Dispositif selon la revendication 1, **caractérisé en ce que** les segments (8) contenant le matériau de remplissage poreux présentent une longueur allant de 0,5 à 10 mm et un diamètre allant de 0,5 à 5 mm.

**3.** Dispositif selon la revendication 1 et/ou 2, **caractérisé en ce que**, au moins, deux dispositifs sont connectés l'un à l'autre.

**4.** Dispositif selon au moins l'une des revendications 1 à 3, **caractérisé en ce que** l'unité d'évaluation comporte un ensemble de photodiode/diode émettrice de lumière permettant d'établir le facteur de transmission.

**5.** Dispositif selon au moins l'une des revendications 1 à 4, **caractérisé en ce que** l'unité d'évaluation comporte un ensemble de diodes émettrices de lumière et une ligne CCD ou une matrice CCD pour établir le facteur de transmission.

**6.** Dispositif selon au moins l'une des revendications 1 à 5, **caractérisé en ce que** le la coloration est effectuée par une conjugaison récepteur-particule.

**7.** Dispositif selon au moins l'une des revendications 1 à 6, **caractérisé en ce que** la coloration est effectuée par une conjugaison récepteur-enzyme suivie de l'addition d'un substrat d'enzyme.

**8.** Dispositif selon les revendications 1 à 7, **caractérisé en ce que** la concentration en récepteur dans les segments augmente de l'orifice d'entrée (3) à l'orifice de sortie (4).

**9.** Procédé pour réaliser des essais quantitatifs rapides par affinité chromatographique pour la présence d'éléments à analyser utilisant une évaluation photométrique dans lequel une solution contenant ou supposée contenir un élément à analyser est introduite par l'orifice d'entrée du dispositif selon l'une quelconque des revendications 1 à 8 et mise en contact avec le matériau de remplissage poreux dans les segments (8) et, après l'adsorption de l'élément à analyser par les récepteurs présentant une affinité pour ledit élément à analyser, la présence de l'élément à analyser lié aux récepteurs est mesurée quantitativement et/ou qualitativement.

Fig.1

Fig.3

Fig.2

ABICAP

ABICAP Multilayer

Multilayer OD sum

**Fig.4**

a           b

f.tularenis      y.pestis

Inlet

Outlet

**Fig.5**

low   middle   high

**Fig.6**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4943522 A
- EP 0557288 A
- EP 0634015 A
- WO 9519569 A
- DE 102004006470 A1
- WO 8707384 A
- EP 0021214 A

**Non-patent literature cited in the description**

- **GESSLER et al.** *Appl. Environ. Microbiol.,* 2005, 7897-7903
- *Appl. Environ. Microbiol.,* 2005, 7897-7903